# EUROPÄISCHE PATENTANMELDUNG

(11) **EP 2 394 693 A1**
(43) Veröffentlichungstag der Anmeldung: **14.12.2011**
(21) Anmeldenummer: 10005994.8
(22) Anmeldetag: 10.06.2010
(51) Int. Cl.: A61N 1/05

(54) **Einrichtung zur Elektroimpulsstimulation der Wunderheilung**

(71) Anmelder: Golsen Limited, 3041 Limassol (CY)
(72) Erfinder: Protsenko, Igor E., 107031 MOSKAU (RU); Boltaev, Anatoly P., 117574 MOSKAU (RU); Pudonin, Fedor A., 142190 g. TROIZK (RU)
(74) Vertreter: Jeck, Anton

(57) **Zusammenfassung**

Die Erfindung betrifft eine Einrichtung der Elektroimpulsstimulation der Wundheilung und von lokalen Entzündungsvorgängen, wobei die Einrichtung einen Stromerzeuger, eine Stromversorgungseinheit, eine Schaltuhr, eine Fußtaste, eine elektronische Steuereinheit und eine Einrichtung zur Förderung der Einwirkungsmenge vom Stromerzeuger zum biologischen Gewebe aufweist. Nach der Erfindung ist ein HF-Erzeuger als Einwirkungsenergieerzeuger eingesetzt, um die Effektivität des Behandlungsvorgangs zu erhöhen, die Heilungsdauer zu verkürzen und die Behandlung von großen Wundflächen sowie von unterschiedlichen Formen von lokalen Entzündungsvorgängen zu beschleunigen.

## Beschreibung

Die Erfindung betrifft eine Einrichtung zur Elektroimpulsstimulation der Wundheilung. Die Erfindung ist in der Medizintechnik einsetzbar. Sie kann zur Behandlung von traumatischen und postoperativen Wunden eingesetzt werden. Dazu zählen auch infizierte Wunden sowie verschiedene Formen von lokalen Entzündungsvorgängen.

Aus dem Patent SU 525961A1 ist eine Mehrkanaleinrichtung bekannt, welche zur Behandlung von Entzündungsvorgängen nach dem Elektropunkturverfahren angewendet wird. Die Einrichtung weist eine elektrische Stromerzeugungseinheit, eine Stromversorgung und eine elektrische Stromschaltereinheit auf, die mit Arbeitseinstichelektroden verbunden sind. Der Mangel dieser Einrichtung ist eine geringe Effektivität des Behandlungsvorgangs sowie eine beachtliche Behandlungsdauer, die damit zusammenhängt, dass die Behandlungsprozedur mehrmals wiederholt werden muss.

Die aus dem Patent RU 2283632C1 bekannte Einrichtung zur chirurgischen Behandlung von Entzündungsvorgängen eines epithelialen Pilonidalsinus (Pilonidalzyste) ist als Prototyp der vorgeschlagenen Einrichtung gewählt. Die Einrichtung weist einen Halbleiterlaser, eine Stromversorgung und eine Steuereinheit, eine Schaltuhr, eine Fußtaste und ein System zur Lichtwellenleiterförderung einer Laserstrahlung in die Einwirkungszone auf. Der Halbleiterlaser funktioniert im Dauerbetrieb mit einer Strahlungswellenlänge von 970 nm und mit einer Leistung von bis zu 2,5 W. Die Funktion der Einrichtung besteht in einer Laserbehandlung der Taschenhöhle und der Fistelgänge einschließlich der Reinigung und der Verdampfung von devitalisierten und modifizierten biologischen Geweben. Die Behandlung erfolgt mittels einer 2- bis 3fachen Gewebe-Laserthermotherapie. Als Mittel für die Beförderung der Laserstrahlung an die Bestrahlungsstelle dient ein flexibler Faserlichtleiter. Sein Distalende wird in den Fistelgang über primäre Öffnungen oder transkutan eingeführt. Die Eintauchtiefe des Distalendes des Lichtwellenleiters wird mittels Ultraschalls überwacht. Die Lasereinwirkungsdauer liegt im Bereich von 30-180 s.

Die Nachteile dieser Einrichtung sind wie folgt:
- eine zu komplizierte Behandlung der großen Wundfläche,
- ein Bedarf von vielen Strahlungsbehandlungen,
- eine individuelle Empfindlichkeit des Patienten gegenüber der Prozedur,
- Schwierigkeiten bei der Optimierung der Bestrahlungsdosis in Bezug auf die Zeit, Fläche und Tiefe der Einwirkung für den jeweiligen Patienten.

Dies vermindert die Effektivität des Behandlungsvorgangs und verlängert die Wundheilungsdauer während der postoperativen Phase.

Es ist Aufgabe der Erfindung, eine Einrichtung zu entwickeln, die Folgendes sicherstellt:
- eine Erhöhung der Effektivität des Behandlungsvorgangs,
- eine Beschleunigung der Heilungsdauer und
- die Möglichkeit der Behandlung von großen Wundflächen und von unterschiedlichen Formen an lokalen Entzündungsvorgängen (z. B. für eine Wundheilung im Perianal- und im Sakral-Steißbeinbereich während einer postoperativen Phase bei den Kranken, welche im Zusammenhang mit Mastdarmfisteln mit einer unterschiedlichen Lokalisation sowie mit chronischen Entzündungen des epithelialen Pilonidalsinus (Pilonidalzyste), Pyodermien usw. operiert worden sind).

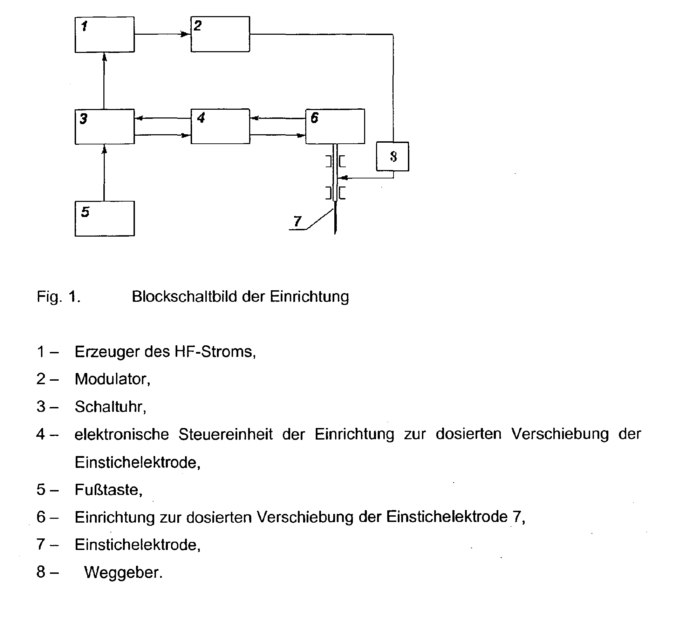

Die gestellte Aufgabe wird durch die Merkmale des Anspruchs 1 gelöst.
Die Erfindung wird anhand eines in Fig.1 gezeigten Schaltbildes näher erläutert.
Die Einrichtung für die Ausführung des vorgeschlagenen Verfahrens (s. Fig. 1) enthält einen Hochfrequenzspannungserzeuger 1, einen Modulator 2 zur Sicherstellung eines erforderlichen Impulstastverhältnisses einer HF-Spannung, eine Schaltuhr 3, eine Fußtaste 5, eine Einrichtung 6 zur dosierten Verschiebung einer Einstichelektrode 7 mit einem Weggeber 8, eine elektronische Steuereinheit 4 zur Steuerung einer dosierten Verschiebung der Einstichelektrode 7.

Die Funktionsweise der Einrichtung ist wie folgt.
Bei der Betätigung einer Fußtaste 5 kommt ein Signal in eine Schaltuhr 3. Von der Schaltuhr 3 aus kommt das Signal in eine elektronische Steuereinheit 4 zur Steuerung einer Vorrichtung 6 zur dosierten Verschiebung einer Einstichelektrode 7. Anhand dieser Vorrichtung 6 ist eine dosierte Verschiebung der Einstichelektrode 7 und ihre Verlagerung auf eine vorgegebene Tiefe sichergestellt. Nach der Einführung der Einstichelektrode 7 in das biologische Gewebe ist das Signal automatisch von der Schaltuhr 3 und einen Weggeber 8 für den Start eines HF-Erzeugers 1 gegeben. Der von diesem Erzeuger 1 fließende Strom ist durch einen Modulator 2 moduliert. Dieser Strom wird an die Einstichelektrode 7 angelegt. Nach Ablauf einer voreingestellten Einwirkungszeit (i - 10 Sek.) des HF-Stroms schaltet die Schaltuhr 3 den HF-Erzeuger 1 aus. Danach wird die Einstichelektrode 7 automatisch aus dem biologischen Gewebe gezogen.

Kenndaten des HF-Erzeugers 1:
Arbeitsfrequenz 100 kHz - 24 MHz
Modulationsfrequenz 0,5 - 10 kHz
Impuls-Pausen-Verhältnis 1 - 30 %
Ausgangsspannungsamplitude 120 - 300 V

Die Kenndaten der Einrichtung des elektromechanischen Moduls 2 zur dosierten Verschiebung der Einstichelektrode 7:
- eine lineare Verschiebung der Einstichelektrode 7 im Bereich von 0 - 15 mm;
- eine Verschiebungsgeschwindigkeit (Eingang - Ausgang) der Einstichelektrode 7 im Bereich von 0,1 - 2 cm/Sek.

Die vorgeschlagene Einrichtung funktioniert in zwei unabhängigen Betriebsarten.

### 1. Betriebsart

Das elektromechanische Modul 2 zur dosierten Verschiebung der Einstichelektrode 7 wird auf der Wundfläche angeordnet. Die Arbeitsfrequenz des HF-Erzeugers 1 ist aus dem Frequenzbereich 1 - 24 MHz, das Impuls-Pausen-Verhältnis aus dem Bereich 1 - 30 %, die Impulsfolgefrequenz aus dem Bereich 0,5 - 10 kHz, die Einwirkungszeit des HF-Stroms aus dem Bereich 3 - 10 Sekunden, die Spannungsamplitude aus dem Bereich 120 - 200 V eingestellt. Die Fußtaste 5 wird betätigt. Das Signal von der Fußtaste 5 ist an die Schaltuhr 3 gesandt. Von der Schaltuhr 3 aus kommt das Signal in die elektronische Steuereinheit 4 der Einrichtung 6 zur dosierten Verschiebung der Einstichelektrode 7. Von hier aus wird das Signal für den Start des elektromechanischen Moduls 2 zur dosierten Verschiebung der Einstichelektrode 7 ausgegeben. Die Verschiebung der Einstichelektrode 7 fängt an. Die Einstichelektrode 7 wird in das biologische Gewebe normal zur Wundfläche auf die vorgegebene Tiefe von 1 - 15 mm eingeführt. Nach der Einführung der Einstichelektrode 7 in das biologische Gewebe gibt die Schaltuhr 3 automatisch ein Signal für den Start des HF-Erzeugers 1 aus. Der durch den Modulator 2 modulierte Strom des HF-Erzeugers 1 wird an die Einstichelektrode 7 angelegt. Nach dem Ablauf der voreingestellten Einwirkungszeit von 1 - 10 Sek. des HF-Stroms schaltet die Schaltuhr 3 den HF-Erzeuger 1 aus. Danach wird die Einstichelektrode 7 automatisch aus dem biologischen Gewebe entfernt. Danach wird das elektromechanische Modul 2 zur dosierten Verschiebung der Einstichelektrode 7 zur neuen Stelle verschoben. Die oben beschriebene Prozedur wird wiederholt, bis die gesamte große Wundfläche behandelt ist.

### 2. Betriebsart

Das elektromechanische Modul 2 zur dosierten Verschiebung der Einstichelektrode 7 wird auf der Wundfläche angeordnet. Das Impuls-Pausen-Verhältnis wird aus dem Bereich 1 -10%, die Impulsfolgefrequenz aus dem Bereich 0,5 - 2 kHz, die Spannungsamplitude aus dem Bereich 200 - 300 V gewählt. Die Fußtaste 5 wird betätigt. Das Signal von der Fußtaste 5 kommt in die elektronische Steuereinheit 4 der Einrichtung 6 zur dosierten Verschiebung der Einstichelektrode 7. Von hier aus wird das Signal zum Starten des elektromechanischen Moduls 2 zur dosierten Verschiebung der Einstichelektrode 7 ausgegeben. Die Verschiebung der Einstichelektrode 7 fängt an. Die Einstichelektrode 7 wird in das biologische Gewebe normal zur Wundfläche auf die vorgegebene Tiefe im Bereich von 4 - 15 mm eingeführt. Nach der Einführung der Einstichelektrode 7 auf die vorgegebene Tiefe ist die Schaltuhr 3 aktiviert. Die HF-Spannung ist an die Einstichelektrode 7 angelegt. Gleichzeitig fängt die Einstichelektrode 7 an, aus dem biologischen Gewebe auszufahren, ohne dass die HF-Spannung abgeschaltet wird. Die Ausfahrgeschwindigkeit der Einstichelektrode 7 aus dem biologischen Gewebe wird im Bereich von 0,1 - 1,5 cm/Sek. eingestellt. In dieser Betriebsart stellt die Einstichelektrode 7 eine Metallnadel mit einem Durchmesser bis zu 0,7 mm dar. Die Nadel ist mit einem Isolierstoff beschichtet und weist einen unisolierten distalen 1 - 2 mm langen Arbeitsteil auf. Die Abschaltung der HF-Spannung erfolgt automatisch nach dem Signal des Weggebers 8, wenn die Einführungstiefe der Einstichelektrode 7 in das biologische Gewebe von 1 - 2 mm erreicht ist. Danach wird das elektromechanische Modul 2 zur dosierten Verschiebung der Einstichelektrode 7 auf eine neue Stelle verschoben. Die beschriebene Prozedur wird wiederholt, bis die ganze Wundfläche behandelt ist. In diesem Fall wirkt die angemeldete Einrichtung auf das biologische Gewebe im Plasma-Mode ein.

Zur Beschleunigung der Behandlung der Wundfläche kann eine Einstichelektrodeeinheit (der Applikator) bis zu 9 Nadeln enthalten. An jede der Nadeln wird die HF-Spannung entweder abwechselnd bzw. gleichzeitig angelegt. Die Einstichelektroden 7 werden aus der Einheit der Einrichtung 6 zur dosierten Verschiebung der Einstichelektroden 7 voneinander getrennt sowohl einzeln als auch gruppenweise (vorgegeben durch das Bedienungspersonal) aus der elektronischen Steuereinheit 6 ausgefahren.

Ein solcher Aufbau des Mehrnadelapplikators ermöglicht es, die Dauer der Durchführung der elektromedizinischen Prozedur wesentlich zu verkürzen.

### Klinisches Beispiel:

Kranke A., 47 Jahre, Krankengeschichte Nr. 482-09. Beschwerden über Eiterfluss aus dem After, Schmerzen im Analkanal beim Stuhlgang.

Bei einer äußeren Betrachtung des Perianalbereichs ist der After zusammengeschlossen, der Perianalreflex ist vorhanden. In der 6-Uhr-Position lässt sich eine trichterförmige Einziehung - eine Außenfistelöffnung - mit einem Durchmesser von ca. 2 mm erkennen. Bei einer Fingeruntersuchung des Mastdarms sind der Sphinktertonus und die Anstrengungen befriedigend. In der 6-Uhr-Position im Bereich der Hinterkrypte lässt sich eine narbengeänderte Innenfistelöffnung mit einem Durchmesser bis zu 2 mm erkennen. Die Untersuchung mit einer Knopfsonde ergibt einen transsphinktären Fistelgang.

Der Kranken wurde die Diagnose - transsphinktäre Hinterfistel des Mastdarms - gestellt.
Die Kranke wurde am 12.12.09 operiert. Die Operation umfasste eine Fistelausschneidung und eine Eröffnung und Drainage des Zwischenafteranlaufens. Am 2. Tag nach der Operation wurde die Patientin mit einer Elektroimpulsstimulation der Wunde nach dem vorgeschlagenen Verfahren behandelt. Es wurde eine mehrfache Einstichelektrode mit zwei nadelförmigen Elektroden eingesetzt. Die Frequenz des HF-Stroms betrug 2,64 MHz, das Impulstastverhältnis 8, die Spannung 160 V, die durchschnittliche Leistung 5 W, die Einwirkungszeit pro Einstich betrug 6 Sekunden. 5 cm² der Oberfläche wurden nach diesem Verfahren behandelt.

Die postoperative Periode verlief ohne Komplikationen. Am 3. Tag nach der Behandlung wurden Ansätze einer Randepithelisation festgestellt. Am 9. Tag nach der Operation wurde eine Wundreinigung mit einer vollständigen Ausbildung eines Granulationsgewebes beobachtet. Gegen den 14. Tag wurde eine Verringerung der Dimensionen der postoperativen Wunde beobachtet. Am 20. Tag wurde makroskopisch eine vollständige Epithelisation der Wunde unter Ausbildung einer Operationsnarbe festgestellt.

Es wird in der Regel eine einmalige Behandlung am 2. Tag nach dem Operationseingriff verordnet. In komplizierten Fällen wird eine Behandlung bis zu dreimal mit einem Abstand von 3 bis 4 Tagen wiederholt.

Somit ermöglicht die vorgeschlagene Einrichtung gegenüber den bekannten Einrichtungen Folgendes:
- die Dauer der Wundheilung nach den Operationen im Zusammenhang mit Fisteln des Mastdarms und der chronischen Entzündung des epithelialen Pilonidalsinus (Pilonidalzyste) zu vermindern, und zwar durch eine Beschleunigung und einen gleichzeitigen Verlauf der Wundprozessphasen;
- die Anzahl der Pflegetage nach der Operation zu verringern und
- die Dauer der sozialen und Arbeitsrehabilitation zu verkürzen.

## Patentansprüche

1. Einrichtung zur Elektroimpulsstimulation der Wundheilung und von lokalen Entzündungsvorgängen, einschließlich eines Stromerzeugers (1), einer Stromversorgungseinheit, einer Schaltuhr (3), einer Fußtaste (5), einer elektronischen Steuereinheit der Einrichtung sowie einer Einrichtung zur Förderung der Einwirkungsenergie vom Stromerzeuger (1) zum biologischen Gewebe,
**dadurch gekennzeichnet,**
**dass** als Einwirkungsenergieerzeuger ein HF-Erzeuger (1) eingesetzt ist, um die Effektivität des Behandlungsvorgangs zu erhöhen, die Heilungsdauer zu verkürzen, die Behandlung von großen Wundflächen sowie von unterschiedlichen Formen lokaler Entzündungsvorgänge zu beschleunigen (z. B. für die Wundheilung im Perianal- und Sakral-Steißbeinbereich während einer postoperativen Phase bei den Kranken, die im Zusammenhang mit Fisteln des Mastdarms mit verschiedener Lokalisierung sowie mit chronischer Entzündung des epithelialen Pilonidalsinus (Pilonidalzyste), Pyodermien usw. operiert wurden).

2. Einrichtung nach Anspruch 1,
**dadurch gekennzeichnet,**
**dass** sie einen Modulator (2) der HF-Spannung aufweist.

3. Einrichtung nach Anspruch 1,
**dadurch gekennzeichnet,**
**dass** sie ein Werkzeug in Form der Gesamtheit der Einstichelektroden (7) zur Einwirkung des Hochfrequenzstroms auf das biologische Gewebe aufweist.

4. Einrichtung nach Anspruch 1,
**dadurch gekennzeichnet,**
**dass** sie eine elektromechanische Einheit (6) zur dosierten Verschiebung der Einstichelektroden (7) aufweist.

5. Einrichtung nach Anspruch 4,
**dadurch gekennzeichnet**,
das sie einen Weggeber (8) der Einstichelektroden (7) aufweist.

6. Einrichtung nach Anspruch 1,
**dadurch gekennzeichnet,**
**dass** der Erzeuger (1) der HF-Spannung eine HF-Spannung mit einer Frequenz von 100 kHz - 24 MHz generiert.

7. Einrichtung nach Anspruch 1,
**dadurch gekennzeichnet,**
**dass** der Erzeuger (1) der HF-Spannung eine HF-Spannung mit einer Amplitude von 120 V bis zu 300 V generiert.

8. Einrichtung nach Anspruch 2,
**dadurch gekennzeichnet,**
**dass** der Modulator (2) die HF-Spannung mit einer Frequenz von 0,5 kHz bis zu 10 kHz moduliert.

9. Einrichtung nach Anspruch 2,
**dadurch gekennzeichnet,**
**dass** der Modulator (2) die HF-Spannung mit einem Impuls-Pausen-Verhältnis von 1%-30% moduliert.

10. Einrichtung nach Anspruch 3,
**dadurch gekennzeichnet,**
**dass** die Einstichelektroden (7) einen Durchmesser bis zu 0,7 mm haben.

11. Einrichtung nach Anspruch 3,
**dadurch gekennzeichnet,**
**dass** die Einstichelektroden (7) bis zu 15 mm lang sind.

12. Einrichtung nach Anspruch 4,
**dadurch gekennzeichnet,**
**dass** sie eine lineare Verschiebung der Einstichelektroden (7) im Bereich von 0 - 15 mm sicherstellt.

13. Einrichtung nach Anspruch 4,
**dadurch gekennzeichnet,**
**dass** die Geschwindigkeit der linearen Verschiebung der Einstichelektrode (7) im Bereich von 0,1 cm/Sek. - 1,5 cm/Sek. liegt.

14. Einrichtung nach Anspruch 5,
**dadurch gekennzeichnet,**
**dass** eine Einspeisung der Einstichelektroden (7) mit der HF-Spannung automatisch nach einem Signal des Weggebers (8) aufhört, wenn die Eintauchtiefe der Einstichelektroden (7) in das biologische Gewebe 1 - 2 mm beträgt.

15. Einrichtung nach Anspruch 1,
**dadurch gekennzeichnet,**
**dass** sie zwei Einwirkungsarten auf die biologischen Gewebe ausführt, wobei sich die Einwirkungsarten je nach der Amplitude der HF-Spannung, die an die Einstichelektroden (7) angelegt ist, unterscheiden.

16. Einrichtung nach Anspruch 15,
**dadurch gekennzeichnet,**
**dass** die Amplitude der HF-Spannung bei der ersten Betriebsart im Bereich von 120 - 200 V liegt.

17. Einrichtung nach Anspruch 16,
**dadurch gekennzeichnet,**
**dass** die Einstichelektroden (7) keine Isolationsbeschichtung haben.

18. Einrichtung nach Anspruch 16,
**dadurch gekennzeichnet,**
**dass** die Übertragung der HF-Spannung auf die Einstichelektroden (7) nach ihrer Einführung in das biologische Gewebe auf die vorgegebene Tiefe erfolgt.

19. Einrichtung nach Anspruch 16,
**dadurch gekennzeichnet,**
**dass** die HF-Spannung in den Einstichelektroden (7) nach Ablauf des auf der Schaltuhr (3) eingestellten Zeitintervalls abschaltbar ist.

20. Einrichtung nach Anspruch 16,
**dadurch gekennzeichnet,**
**dass** die Einstichelektroden (7) aus dem biologischen Gewebe automatisch nach der Abschaltung der HF-Spannung ausfahrbar sind.

21. Einrichtung nach Anspruch 15,
**dadurch gekennzeichnet,**
**dass** die Amplitude der HF-Spannung in der 2. Betriebsart im Bereich von 200 - 300 V liegt.

22. Einrichtung nach Anspruch 21,
**dadurch gekennzeichnet,**
**dass** die Einstichelektroden (7) eine Isolationsbeschichtung aufweisen, wobei ihr Distalteil unisoliert bleibt.

23. Einrichtung nach Anspruch 22,
**dadurch gekennzeichnet,**
**dass** die Einstichelektroden (7) einen unisolierten distalen 1 - 2 mm langen Arbeitsabschnitt aufweisen.

24. Einrichtung nach Anspruch 21,
**dadurch gekennzeichnet,**
**dass** die Einstichelektroden (7) unter HF-Spannung setzbar sind, sobald das Herausführen der Einstichelektroden (7) aus dem biologischen Gewebe beginnt.

25. Einrichtung nach Anspruch 3,
**dadurch gekennzeichnet,**
**dass** die Einstichelektrodeeinheit bis zu 9 aufweist.

26. Einrichtung nach Anspruch 4,
**dadurch gekennzeichnet,**
**dass** die Einstichelektroden (7) aus der Einheit der Einrichtung (6) zur dosierten Verschiebung der Einstichelektroden (7) voneinander getrennt ausfahrbar sind.
